# EUROPEAN PATENT APPLICATION

(11) **EP 2 314 346 A1**
(43) Date of publication of application: **27.04.2011**
(21) Application number: 10157079.4
(22) Date of filing: 19.03.2010
(51) Int. Cl.: A61N 1/36

(54) **Method and apparatus for treatment of neurodegenerative diseases including depression, mild cognitive impairment, and dementia**

(30) Priority: 23.10.2009 US 604701
(71) Applicant: Neuromed Devices, Inc., Oakley, UT 84055 (US)
(72) Inventor: Silverstone, Leon M, Oakely, UT UT84055 (US)
(74) Representative: Purdy, Hugh Barry

(57) **Abstract**

A method and apparatus for treatment of neurodegenerative diseases including depression, mild cognitive impairment and dementia. An electrical signal comprising a stimulation frequency is generated and applied transcutaneously to a patient over at least one treatment cycle comprising a plurality of intervals, wherein each interval comprises increasing the stimulation frequency to a high frequency and decreasing the stimulation frequency to a low frequency.

## Description

### BACKGROUND OF THE INVENTION

### FIELD OF THE INVENTION

Embodiments of the invention described herein pertain to the field of neuromodulation. More particularly, but not by way of limitation, one or more embodiments of the invention enable a method and apparatus for treatment of neurodegenerative diseases including depression, mild cognitive impairment, and dementia.

### DESCRIPTION OF THE RELATED ART

The human brain is made up of a trillion cells, one hundred billion of them being neurons linked in a vast network. Neurons receive signals on highly branched extensions of their bodies called dendrites and send the information along a single extension called an axon. Axons are microscopic in size but can be up to two meters in length, and branch into multiple terminals that synapse with many different cells. Communication along individual nerve cells occurs by electrical impulses called action potentials. Communication between nerve cells occurs at synaptic junctions, where neurotransmitters are transferred from one neuron to another neuron or to a different cell type. There can be as many as ten thousand synaptic junctions along a single neuron.

Brain neurochemicals are essential for all quality of life events such as memory, sleeping, not feeling depressed, not over-eating, and the ability to control pain and prevent degenerative diseases. Although the brain can manufacture these neurochemicals in adequate amounts early on in life, their supply becomes reduced as an individual ages beyond its 20's and 30's and so on. The end result is an increased susceptibility to aging as well as to debilitating diseases, such as, for example, Parkinson's disease as well as others linked to neurochemical insufficiencies. In addition, reduced neurochemical levels are a significant etiological factor in the development of mild cognitive impairment (MCI) leading to dementia and Alzheimer's disease.

Neurochemical modulation results in nociceptor activity whereby the production of neurotransmitters in the nucleus of presynaptic cells is enhanced. There are three stages in the process of chemical production and transference to the adjacent cell. Once produced, neurotransmitter molecules are stored in synaptic vesicles, which diffuse along the single neural axon to presynaptic junctions. This constitutes stage one in the sequence. As a result of firing of action potentials, calcium ions enter the pre-synaptic cell to initiate neurotransmitter release. This occurs in multi-molecular pockets or quanta (thousands of molecules) by exocytosis in which synaptic vesicles fuse with the pre-synaptic membrane, thereby releasing neurotransmitters into the synaptic cleft (gap between the pre and post synaptic junction). In addition, release of secondary chemical messengers result in ion channels opening in the post-synaptic membrane to allow uptake of neurotransmitter molecules. This constitutes stage two in the sequence. Reuptake of neurotransmitter molecules, which were not taken up by the post-synaptic membrane, occurs in the pre-synaptic membrane, a condition referred to as endocytosis, which is the reverse of the process of exocytosis. This latter sequence constitutes stage three in the sequence.

It is interesting to note that as a result of taking SSRI antidepressant drugs, stage three of this dynamic sequence, neurotransmitter re-uptake (*e.g*. endocytosis) is inhibited, leading to untoward clinical effects. In 2002 about 11 million prescriptions of SSRI antidepressants were written for people under 18 years of age. In December 2006, a U.S. Food and Drug Administration (FDA) Advisory Panel warned that the risk of suicide linked to SSRI antidepressants should be extended from children and adolescents, to include adults up to the age of 25 years (Reuters, 2006). The new recommendations came in the wake of an FDA review, released in December 2006, of more than 372 studies of 11 drugs, including Zoloft, Prozac, Paxil and Lexapro. In May 2007, The Food and Drug Administration ordered drug makers to add warnings to antidepressant medications, saying the drugs increase the risk of suicidal thinking or behavior in some young adults. The FDA found that the risk for suicide attempts at least doubled in people 25 years and under when they took SSRI antidepressants.

Additionally, the American College of Obstetricians and Gynecologists recommended in the December 2006 issue of Obstetrics & Gynecology that pregnant women and those wishing to become pregnant refrain from taking the SSRI antidepressant Paxil because of the risk for birth defects. In the June 25, 2007, Archives of Internal Medicine, bone density was found to be significantly lower in a group of about 6,000 men aged 65 years and older who had been taking SSRI antidepressants (Haney, E.M. et al, 2007). In the Archives of Internal Medicine, Diem et al (2007) also reported an increased rate of bone loss at the hip in a group of more than 2,700 older women. In both groups, bone loss was only found in those using SSRI antidepressants. Hence the use of drugs that do not replicate the normal physiological events at synaptic junctions can have significant untoward clinical effects.

"Neuromodulation" is a term that has now become a favored term used by drug companies and device manufacturers alike. However, this term is often inaccurately used to describe the mechanisms of action of treatment drugs, such as SSRI antidepressants, that claim they are able to increase the supply of serotonin at synaptic junctions. The term is also used synonymously with "neurostimulation" and "electrical stimulation", descriptions which apply to very different and much earlier device technologies, as well as to devices that are currently implanted in the brain or elsewhere in the body. At the present time, despite claims by some pharmaceutical companies, no drugs can increase the production of naturally synthesized neurotransmitters in the human nervous system. The mechanism of action of drugs, such as the selective serotonin reuptake inhibitor antidepressants (SSRI's), is to inhibit the re-uptake of neurotransmitters that are not taken up by the adjacent cell, a mechanism referred to as inhibiting endocytosis. This non-physiological process has its own clinical complications.

Neurodegenerative disease refers broadly to conditions where neurons are lost. Because cells of the brain and the spinal cord do not regenerate regularly, the damage caused by neurodegenerative disease is cumulative. Dementia refers to a set of symptoms arising from such cumulative damage. Some neurodegenerative disorders, such as Alzheimer's disease, have a genetic component that indicates a person is at higher risk. In Alzheimer's disease, amyloid plaques build up in brain tissue over time, causing increasing symptoms of dementia. Over the past sixteen years scientists have been aware that Epsilon4 allele carriers (the ApoE-4 gene) have a high risk factor for Alzheimer's disease (Saunders et al, 1993). Furthermore the oral herpes virus (Herpes Simplex virus type 1 (HSV-1)), has been identified as a risk factor for Alzheimer's disease when the ApoE-epsilon4 gene is in the central nervous system. Approximately 90% of amyloid plaques in Alzheimer's disease brains were found to contain HSV-1 DNA (Wozniak, Mee and Itzhaki, 2009).

Nociceptors are sensory neurons which react to potentially damaging stimuli. Nociceptors convey pain signals to the brain and spinal cord via Aδ and C nerve fibers to higher brain centers. Aδ fibers are thin axons of neurons insulated with a myelin sheath. Aδ fibers conduct at a moderate velocity and are typically associated with a sharp pain response and temperature sensation. C fibers are unmyelinated axons related to the somatic sensory system with a slow conduction velocity. Neurochemical modulation occurs in response to nociceptor activity. Conventional electrical stimulation devices transmit electrical frequency signals along Aβ nerve fibers.

Only pain signals are able to access and travel along nociceptor nerve pathways. These first order neurons enter the dorsal horn of the spinal cord and synapse with second order neurons. Second order neurons then cross the spinal cord and join with the lateral spinothalamic tract, which carries pain impulses up the spinal cord into the brainstem, midbrain and higher brain centers such as the thalamus and cerebral cortex. The lateral spinothalamic tract then synapses with third order neurons, which ascend to the cerebral cortex. The brainstem and thalamus are responsible for pain perception whereas the cerebral cortex gives pain location and pain intensity.

These pathways are very important in the production of neurochemicals, which play vital roles in quality of life events. The descending inhibitory pathways begin in the cerebral cortex of the higher brain centers and descend to the thalamus and then synapse at the periaqueductal gray of the midbrain. The periaqueductal gray is an important region since it is rich in opiate receptors responsible for secreting morphine-like enkephalins and endorphins. Fibers from the periaqueductal gray then descend and synapse at another very important region of the descending inhibitory pathway, the nucleus raphe magnus, located in the brainstem. The nucleus raphe magnus is responsible for the secretion of 5-hydroxytryptophan (serotonin), which plays important roles in elevating pain threshold, and combating depression and erratic behavior.

Fibers then descend from the nucleus raphe magnus and enter the spinal cord, exciting other inhibitory interneurons to secrete additional powerful anti-pain neurotransmitters such as gamma-aminobutyric acid (GABA). These lower fibers of the descending inhibitory pathways synapse with interneurons, which communicate with pain signals entering the spinal cord via the pain conveying nociceptors, A-δ and C fibers, as well as with the second order neurons of the lateral spinothalamic tract. This demonstrates that the descending inhibitory pathways act at all levels from the higher brain centers down to the dorsal horn region of the spinal cord where pain signals first enter the central nervous system. In addition to the control of pain and depression, neurotransmitters also enhance tissue healing, increase quality of sleep time, reduce appetite and erratic behavior, and generally increase quality of life.

Development of an earlier non-invasive neuromodulation device was used to produce both anesthesia and analgesia for dental treatment, eliminating the use of invasive needles and anesthetic fluids (Silverstone, 1989). Dental pain was considered an ideal test since a placebo effect was unlikely to be significant with procedures such as restoration or extraction of teeth. After obtaining relevant patents (O'Neill, Silverstone and Halleck, 1990) and regulatory clearance, six clinical studies were carried out under the direction of six dental surgeons in Britain during the period 1988-1990. The developer of the device (Silverstone, 1989) was not involved in the studies to eliminate bias. The studies included a total of 825 adult subjects who had restorative dental treatment carried out using the Electronic Dental Anesthesia neuromodulation device. The non-invasive device was powered by two 1.5v D-cell flashlight batteries, had a pulse frequency of 16K Hertz, a peak current of 0.87 milliamps AC, and a peak voltage of 2 volts into a 1000 Ohms test load. Results showed a mean weighted efficacy of 95%, with a range of 89-98% in effectiveness. Dental anesthesia by needle usually produces a mean efficacy of 89%. This technology targeted endorphins and serotonin, both of which were elevated prior to, during, and following operative treatment. In addition to producing highly efficient anesthesia and removing the fear of the needle, it also eliminated the risk of transmission of Hepatitis-B and the AIDS virus (Mann and Silverstone, 1990).

Neuromodulation refers to the regulation of neurons by several classes of neurotramitters in the nervous system. The secretion of neuromodulatory transmitters in one region of the brain affects large areas of the nervous system. The broad effect caused by the diffusion of neuromodulatory transmitters contrasts with the effect of neurotransmitters involved in direct synaptic transmission between a presynaptic neuron and a postsynaptic neuron. Neuromodulatory transmitters include dopamine, epinephrine, norepinephrine, serotonin, acetylcholine, histamine, and other neurotransmitters capable of neuromodulation.

Chemical imbalances in the brain have been associated with conditions such as depression, mild cognitive impairment, and dementia. Pharmacological treatments are commonly used to correct neurochemical imbalances in patients undergoing treatment for depression, mild cognitive impairment, and dementia. It is claimed that these pharmacological compounds affect the release and absorbtion of neuromodulatory transmitters.

Another method often described as providing neuromodulation is direct electrical stimulation. For example, surgically implanted spinal cord stimulators are used for treating neurological pain. Another device commonly referred to as a brain pacemaker is surgically implanted into the brain to provide deep brain stimulation. However, both these methods involve invasive, risky and complex surgical techniques. Senior medical representatives of the companies that manufacture such brain implants refer to the technology during presentation as "electrical stimulation" or "electrical neurostimulation". In spite of the regulatory approval in 1997 of such brain implants for use in the control of essential tremor, there is no evidence that this approach can produce neurochemical modulation.

The implanted electrode emits an electrical signal that neutralizes the inhibitory signal that is apparently the cause of the tremor condition. This is seemingly confirmed since in subjects that have success with the control of one contralateral hand after implantation of the electrode, the positive effect disappears immediately the electrode is turned off. The implants produce a limited signal that is apparently little different from signals employed using routine electrical stimulation technologies that have been employed for decades. When used to control essential tremor, an implant in one thalamus can only stop hand tremor in one hand positioned contralateral to the implant in about 30% of the cases Furthermore, the implant cannot stop bilateral hand tremors or head tremor, as can non-invasive neuromodulation (Silverstone et al, 1998). A recent published study employing implants in both thalami to produce deep brain stimulation in an attempt to control tremors in Parkinson's disease was compared with best medical therapy by the use of pharmaceutical drugs as six months (Weaver et al., 2009). Although producing better outcomes than drugs, forty-nine deep brain stimulation patients (40%) experienced 82 serious adverse events. In contrast, fifteen best medical therapy patients (11%) experienced 19 serious adverse events. In addition to increased risk, patients who received deep brain stimulation lost some verbal fluency, memory, and information processing speed. A further disadvantage of this approach is the high cost of surgery; it costs about $65,000 to implant the electrodes. Once implanted, in the event of negative side effects, even a single implant cannot be removed. The implant has to be turned off by passing an electromagnet over the chest where the pulse generator is implanted behind the clavicle.

Electroshock therapy is a noninvasive method of providing electrical stimulation to treat neurological conditions. Electroshock therapy uses high levels of electrical stimulation to induce seizures, resulting in severe side effects such as memory loss and confusion. The exact mechanism of electroshock therapy has not been determined, but researchers theorize several theories of action, including neurotransmitter effects, anti-convulsant effects, neuroendocrine effects and brain damage. The use of electroshock therapy is limited due to the extreme effects and there is no evidence that this treatment approach can produce neurochemical modulation.

In contrast to the action of some drugs, medical devices that employ "non-invasive therapeutic neuromodulation" as their mechanism of action are able to enhance the synthesis of neurotransmitter molecules within neuronal nuclei. Synaptic vesicles containing these molecules travel along the axon to its pre-synaptic membrane, which are released into the synaptic cleft, and taken up into ion channels which open in the post-synaptic membrane of an adjacent cell. Most importantly, with neurotransmitters that are not taken up by the adjacent cell, reuptake occurs back into the pre-synaptic membrane that originally released them. This mechanism simulates normal physiological events at the neural synapse, as opposed to the non-physiological mechanism of inhibiting reuptake of molecules back into the cell.

As such, there is a need for a neurochemical modulation method and apparatus for safe, non-invasive neurochemical modulation for the treatment of neurodegenerative diseases including depression, mild cognitive impairment, and dementia.

### BRIEF SUMMARY OF THE INVENTION

One or more embodiments of the invention enable a method and apparatus for treatment of neurodegenerative diseases including depression, mild cognitive impairment, and dementia.

An apparatus and methods for treating patient ailments in various aspects of neurological disorders delivers electrical stimulation coupled with alternating high and low frequencies to the skin or mucosa of a patient so as to produce non-invasive therapeutic neuromodulation. Systemic neuromodulation is produced by a series of electrical pulses having various electrical characteristics coupled with alternating frequency outputs. The apparatus includes a housing having at least two electrodes supplied with either or both AC and DC voltages. The AC voltages have various patterns and ranges of output frequencies. Devices will be powered by an internal battery system. Tissue contact electrodes are placed in various body locations in relation to either anatomical, skeletal or energy sites to ensure treatment to all parts of the anatomy.

In one or more embodiments of the invention, non-invasive neurochemical modulation devices, also referred to as non-invasive neuromodulation devices, are transcutaneous electrical nerve and frequency stimulation devices that produce non-invasive therapeutic neuromodulation. This results in electrical-frequency signals being transmitted via nociceptors, that is neurons that convey pain signals via Aδ and C nerve fibers to higher brain centers to provide neurochemical modulation, a unique concept in the otherwise long established electrical stimulation field, rather than just transmission along large "Aβ" nerve fibers as with conventional devices.

Thus, the invention provides an apparatus for providing non-invasive neuromodulation for use in treating a neurological disorder in a patient, the apparatus comprising:
means for generating an electrical signal comprising a stimulation frequency; and
means for applying said electrical signal transcutaneously to a patient over at least one treatment cycle comprising a plurality of intervals, wherein each interval comprises:
   increasing said stimulation frequency to a high frequency; and
   decreasing said stimulation frequency to a low frequency,
   wherein said electrical signal is transmitted by Aδ and C nerve fibers.
Suitably, the apparatus further comprising means for applying at least one additional monotonically decreasing treatment cycle, wherein said at least one additional monotonically decreasing treatment cycle comprises a monotonically decreasing electrical signal beginning at about 150 KHz or greater, wherein said monotonically decreasing electrical signal is reduced over a time period to about 50 Hz or lower without periodic increases in frequency, wherein said time period is from about ten seconds to about sixty minutes. Typically, said neurological disorder is selected from clinical depression, and cognitive impairment. Preferably, said stimulation frequency is variable between .01Hz and 1,000,000 Hz.

The invention also provides an electrical stimulation device comprising a first electrode and a second electrode, wherein said first electrode and said second electrode are configured to apply an electrical stimulation transcutaneously to a patient at a variable stimulation frequency, for use in treating a neurological disorder in a patient by non-invasive neuromodulation, said use comprising the steps of obtaining a treatment profile comprising a series of first frequency settings A₀, A₁, A₂, ..., A_{N}, a series of second frequency settings B₁, B₂, ...,B_{N} and a series of time durations t₁, t₂, ..., t_{2N}, wherein Aᵢ ≤ Aᵢ₋₁ and Bᵢ ≤ Bᵢ₋₁;
applying at least one treatment cycle based on said treatment profile, wherein applying a treatment cycle comprises:
applying said electrical stimulation transcutaneously to a patient, wherein said variable stimulation frequency is set to a frequency A₀;
increasing and decreasing the variable stimulation frequency sequentially over N intervals, wherein increasing and decreasing comprises:
   (a) steadily changing the variable stimulation frequency to said second frequency setting B₁ over time duration t₁,
   (b) steadily changing the variable stimulation frequency to A₁ over time duration t₂,
   (c) repeating steps (a) and (b) sequentially for all first frequency settings and all second frequency settings.

In one embodiment, Aᵢ₋₁ ≤ Bᵢ or Aᵢ₋₁ > Bᵢ. Suitably, said stimulation frequency is variable, typically between .01Hz and 1,000,000 Hz. In a preferred embodiment, the use further comprises applying at least one additional monotonically decreasing treatment cycle, wherein said at least one additional monotonically decreasing treatment cycle comprises a monotonically decreasing electrical signal beginning at about 150 KHz or greater, wherein said monotonically decreasing electrical signal is reduced over a time period to about 50 Hz or lower without periodic increases in frequency, wherein said time period is from about ten seconds to about sixty minutes. Typically, said treatment profile is designed for the treatment of clinical depression and/or cognitive impairment. Suitably, the use further comprises obtaining a second treatment profile, and applying at least one treatment cycle based on said second treatment profile.

In embodiment, the use further comprises applying a modified treatment cycle based on said treatment profile, wherein modified durations t'₁, t'₂, ..., t'_{2N} are scaled based on said durations t₁, t₂, ..., t_{2N} .

In one embodiment, the use further comprises applying a modified treatment cycle based on said treatment profile, wherein a modified series of first frequency settings and a modified series of second frequency settings are scaled based on said series of first frequency settings and said series of second frequency settings and/or said at least one treatment cycle comprises at least two treatment cycles and said at least two treatment cycles are applied at different voltage intensities.

The invention also provides a device for providing non-invasive neuromodulation to treat a neurological disorder, comprising:
a housing;
at least two electrodes configured to apply an electrical stimulation at a variable stimulation frequency;
a power source;
a treatment program stored in a memory comprising a treatment profile, said treatment profile comprising a series of first frequency settings A₀, A₁, A₂, ..., A_{N}, a series of second frequency settings B₁, B₂, ...,B_{N} and a series of time durations t₁, t₂, ..., t_{2N}, wherein Aᵢ ≤ Aᵢ₋₁ and Bᵢ ≤ Bᵢ₋₁;
an electronic circuit positioned inside said housing and coupled to said power source,
said memory and said at least two electrodes, wherein said electronic circuit is configured to execute said treatment program, wherein executing said treatment program comprises:
   generating said electrical stimulation;
   setting said variable stimulation frequency to A₀;
   increasing and decreasing the variable stimulation frequency sequentially over N intervals, wherein increasing and decreasing comprises
      (a) steadily changing the variable stimulation frequency to said second frequency setting B₁ over time duration t₁,
      (b) steadily changing the variable stimulation frequency to A₁ over time duration t₂, and
      (c) repeating steps (a) and (b) sequentially for all first frequency settings and all second frequency settings.
   Suitably, said treatment profile is designed for the treatment of clinical depression and/or cognitive impairment. Ideally, said device is a portable device, said power source is a battery, and said at least two electrodes are located on a contact surface coupled with said housing, wherein said contact surface is configured to be applied transcutaneously to a patient.

### BRIEF DESCRIPTION OF THE DRAWINGS

The above and other aspects, features and advantages of the invention will be more apparent from the following more particular description thereof, presented in conjunction with the following drawings wherein:

**FIG. 1** illustrates the circuitry provided inside the housing of a neurochemical modulation device in accordance with one or more embodiments.

**FIG. 2A** illustrates a portable neurochemical modulation device for treating neurodegenerative disorders in accordance with one or more embodiments.

**FIG 2B** illustrates electrodes used with one or more embodiments of a neurochemical modulation device.

**FIG. 3A** illustrates an electrode placement protocol comprising treatment sites based on chakras used with one or more embodiments of a neurochemical modulation device.

**FIG. 3B** illustrates an electrode placement protocol comprising treatment sites based on the Central Location used with one or more embodiments of a neurochemical modulation device.

**FIG. 4** presents a graph of the frequency sweep over an exemplary treatment cycle generated from the treatment profile of Table 1, wherein the frequency is adjusted linearly over each time period.

**FIG. 5** presents a graph of the frequency sweep of an exemplary treatment cycle generated from a treatment profile for the treatment of clinical depression.

**FIG. 6** presents a graph of the frequency sweep of an exemplary treatment cycle generated from a treatment profile for the treatment of mild cognitive impairment and dementia.

**FIG. 7** presents a graph of neurochemical levels in patients before treatment, 20 minutes into treatment, and 24 hours after treatment of patients with a neurochemical modulation device in accordance with embodiments of the invention.

### DETAILED DESCRIPTION

A method and apparatus for treatment of neurodegenerative diseases including depression, mild cognitive impairment, and dementia will now be described. In the following exemplary description numerous specific details are set forth in order to provide a more thorough understanding of embodiments of the invention. It will be apparent, however, to one of ordinary skill in the art that the present invention may be practiced without incorporating all aspects of the specific details described herein. In other instances, specific features, quantities, or measurements well known to those of ordinary skill in the art have not been described in detail so as not to obscure the invention. Readers should note that although examples of the invention are set forth herein, the claims, and the full scope of any equivalents, are what define the metes and bounds of the invention.

**Non-invasive neuromodulation Device**

An apparatus and methods for treating neurodegenerative disorders delivers electrical stimulation at high frequencies so as to produce non-invasive therapeutic neuromodulation. The electrical stimulation is delivered transcutaneously, such as through the skin or mucosa of a patient.

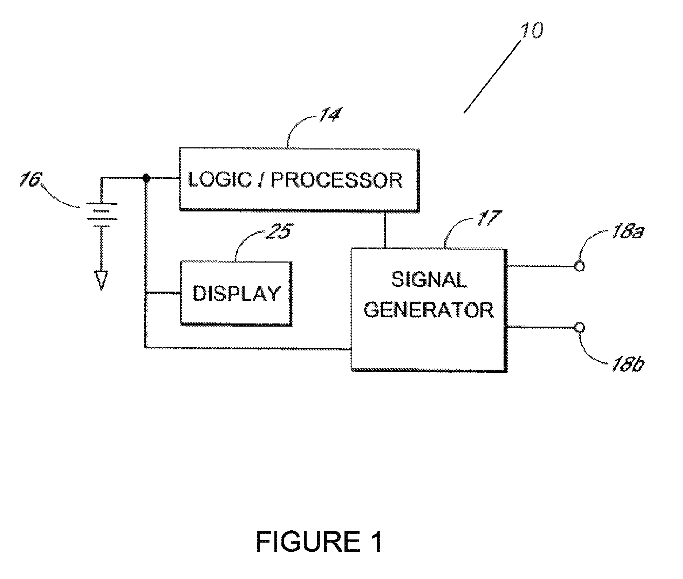

FIG. 1 illustrates the circuitry provided inside the housing of a non-invasive neuromodulation apparatus in one or more embodiments of the invention. Non-invasive neuromodulation device 10 includes battery 16. High-tech internal battery systems known in the art are compatible with embodiments of the non-invasive neuromodulation device. Battery access is preferably simple so that batteries can be replaced in the field. The noninvasive neuromodulation device 10 may use rechargeable batteries. The usage of appropriate transformers may avoid accidental battery discharge during treatment. In one or more embodiments, an external transformer is employed to maintain charge during treatment, allowing for recharging while a working battery is in situ in the device. External recharging systems using transformers of different output parameters can be configured such that the recharging system functions internationally. Alternatively, non-portable embodiments of the invention may run on alternate sources of power, such as A/C power and D/C power, including D/C power generated by an A/C to D/C converter.

Battery 16 is coupled to logic/processor circuit 14. Logic/processor circuit 14 drives display 25 of non-invasive neuromodulation device 10. Display 25 visually provides an operator with information regarding the operation of non-invasive neuromodulation device 10. Logic/processor circuit 14 configures a signal generator circuit 17 to output the desired electrical signal outputs 18a and 18b for treating neurodegenerative disorders. Although shown in separate blocks, it is possible to combine elements of logic /processor circuit 14 and signal generator circuit 17 and to print logic/processor circuit 14 and signal generator circuit 17 on a single circuit board. Crystal based digital frequency generation of electrical signal outputs 18a and 18b is preferable for accuracy.

Non-invasive neuromodulation device 10 has a minimum of two independent channels (*e.g*. electrical signal outputs 18a and 18b). Electrical signal outputs 18a and 18b are connected to electrodes which transcutaneously deliver electrical stimulation to a patient. Electrical signal outputs 18a and 18b are of variable frequency and variable amplitude which are controlled by logic/processor circuit 14 based on a treatment program. In one embodiment, the amplitude of electrical signal outputs 18a and 18b ranges from 0 to 60 volts peak to peak with a 500 Ohm .1 µf, load. Alternatively, an AC or DC voltage of 0-5K volts is employed. The frequency of electrical signal outputs 18a and 18b may range from 0.01 Hz to 1,000,000 Hz. Optionally, a maximum charge per pulse is employed by for electrical signal outputs 18a and 18b as a safety feature. A maximum charge ranging from 75-500 microcoulombs is employed in varying embodiments of the device

Electrical signal outputs 18a and 18b may comprise a variety of waveform types which can include symmetrical biphasic waveforms, asymmetrical biphasic waveforms, sinusoidal, rectangular, triangular and spike phase shape AC waveforms. Waveforms may have a fast rise and a slow decay in order to simulate the action potential of human neurons. The outputs can be employed with any single waveform type, or combinations of waveform types.

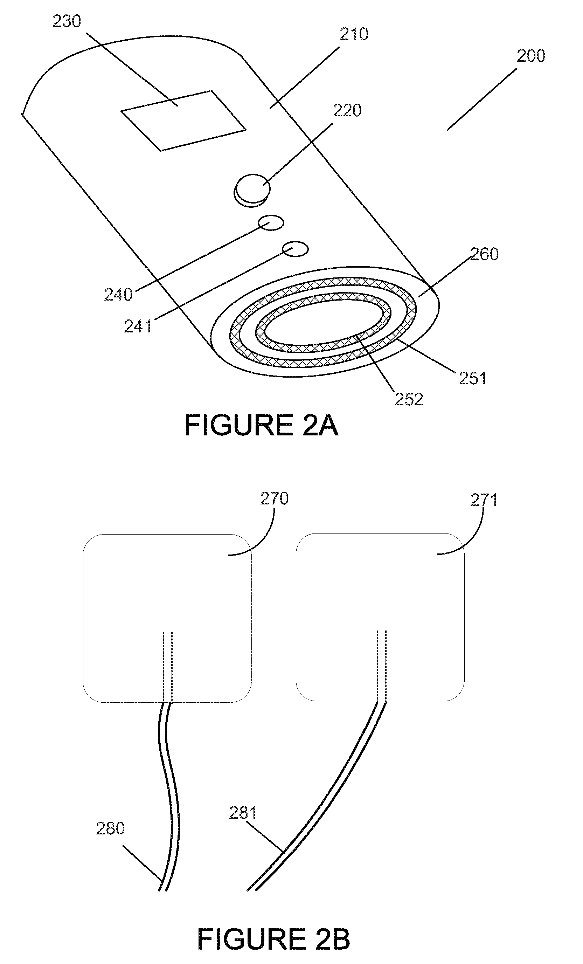

FIG. 2A illustrates a portable non-invasive neuromodulation device for treating neurodegenerative disorders. Device 200 includes housing 210. Housing 210 of device 200 is designed for portability and ease of operation by a user. A user may hold and operate one or more embodiments of device 200 with one hand. The size and shape of housing 210 is dependent on the treatment area device 200 is designed for. Housing 210 is coupled with contact surface 260. Contact surface 260 is configured to provide full contact between device 200 and the skin or mucosa of a patient. For example, contact surface 260 may provide a curved surface matching a contour of a treatment site for a neurodegenerative disorder on a patient. A concave contact surface is suitable for convex treatment sites, while a convex contact surface is suitable for concave treatment sites or treatment sites comprising soft tissue capable of receiving and taking on the curvature of contact surface 260. In one or more embodiments, multiple interchangeable contact surfaces are provided with one device 200.

Electrodes 251-252 are positioned on contact surface 260. Electrodes 251-252 may take many forms and shapes, including rounded and geometric shapes. Electrodes 251-252 are elongated to provide an enlarged contact surface area between electrodes 251-252 and a patient's skin or mucosa. In one or more embodiments, electrodes 251-252 are nested closed contours printed on a circuit board which functions as contact surface 260. Electrodes 251-252 are configured to deliver transcutaneous electrical stimulation to a patient based on electrical outputs generated by the circuitry of device 200. Electrodes 251-252 are composed of or plated with an electrically conductive material, such as gold.

Device 200 further comprises switch 220. Switch 220 is used to manually initiate a treatment cycle. Optionally, switch 220 and additional switches provide additional input mechanisms to a user operating device 200. Switch 220 can be used to initiate early termination of a treatment cycle, selection of a treatment program, creating a treatment program, accessing a treatment history, switching display modes, or any other operational feature.

LEDs 240-241 and display 230 provide information regarding the status and operation of device 200. LEDs 240-241 are useful for indicating a low battery condition, current operation, or any other status of device 200. Display 230 provides alphanumerical or graphical information to the user regarding the status of the device or the status or history of a treatment. Display 230 is optionally a touch-screen display which serves as an additional user input device for the operation of device 200.

Device 200 is operated by a patient receiving the transcutaneous electrical stimulation. Alternatively, another user may operate portable or non-portable embodiments of the device, such as a care provider administrating a treatment to a patient. Although FIG. 2A illustrates a device 200 as a portable device, one of ordinary skill in the art would appreciate that embodiments of the invention include non-portable devices, such as A/C powered devices or larger devices powered by either an A/C or D/C power source. Additionally, contact surface 260 and electrodes 251-252, which serve to provide a contact suitable for delivering transcutaneous electrical stimulation, are replaceable by other components. For example, in FIG. 2B, electrode contact surfaces 270-271 deliver electrical outputs transmitted along wires 280-281, which are configured to couple with the circuitry of the non-invasive neuromodulation device.

**Treatment Sites**

Embodiments of the non-invasive neuromodulation device described in this disclosure are compatible with a number of electrode placement protocols. Electrode placement protocols comprise related treatment sites for electrode placement corresponding to anatomical features and systems. A treatment program may include a subset of treatment sites from an electrode placement protocol, all treatment sites in an electrode placement protocol, or a combination of treatment sites from multiple electrode placement protocols.

In one or more electrode placement protocols, electrodes are placed in relationship to masses of neural tissue including nociceptors. Masses of neural tissue outside of the central nervous system are called neural ganglia. The cell body of a neuron associated with a Aδ nerve fiber is located in a trigeminal ganglion or a dorsal root ganglion.

In one or more electrode placement protocols, treatment sites are based on the location of regional lymph nodes.

In one or more electrode placement protocols, treatment sites are based on the location of air sinuses such as those in the facial bones of the skull. Examples include the maxillary antra positioned bilaterally below the eyes and above the upper teeth, accessed by placement either side of the nose, level with the eyes, and the frontal sinus positioned either side of the midline on the forehead just above the eyes.

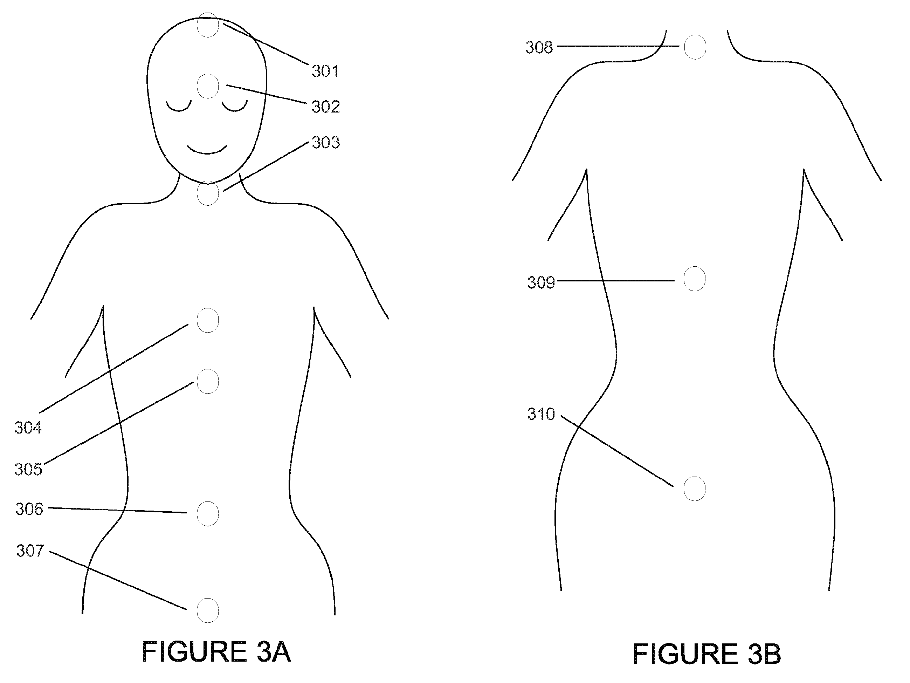

It is theorized that in addition to a network of nerves and sensory organs, there also exists a subtle system of channels and centers of energy (chakras) which affects the physical, intellectual, emotional and spiritual being. **FIG. 3A** illustrates an electrode placement protocol comprising treatment sites based on chakras. Treatment sites 301-307 are located on the ventral position of the body. Treatment site 301 corresponds to the crown chakra, which is associated with the pituitary gland. Treatment site 302 corresponds to the brow chakra, which is associated with the pineal gland. Treatment site 303 corresponds to the throat chakra, which is associated with the thyroid. Treatment site 304 corresponds to the heart chakra, which is associated with the thymus. Treatment site 305 corresponds to the solar plexus chakra, which is associated with the Islets of Langerhans. Treatment site 306 corresponds to the sacral chakra, which is associated with the genitourinary system and the adrenal glands. Treatment site 307 corresponds to the base chakra, which is associated with the adrenal medulla.

"The Central Location" refers to a collection of three different anatomical regions. **FIG. 3B** illustrates an electrode placement protocol comprising treatment sites based on the Central Location. Treatment sites 308-310 are centered around the spine on the dorsal position of the body. Cervical treatment site 308 is located slightly above the level of the shoulders at the seventh cervical vertebra (C7). Thoracic treatment site 309 is positioned between the neck and the base of the spine at the seventh thoracic vertebra (T7). Lumbar treatment site 310 is positioned at the base of the lumbar spine at the fifth lumbar vertebra (L5). Different treatment protocols are carried out on treatment sites 301-307, treatment sites 308-310, treatment sites 301-310 in combination, or any subset of treatment sites 301-310.

**Treatment Programs**

When applied transcutaneously at a treatment site, the electrical signal outputs produce an electrical stimulation treatment program for non-invasive therapeutic neurochemical modulation. Electrical frequency signals are transmitted to higher brain centers via nociceptors, which convey pain signals via Aδ and C nerve fibers. Conventional devices transmit electrical frequency signals along A β nerve fibers.

A treatment program involves frequency, amplitude and time modulation. A user may select a treatment program using a touch-screen display, a switch, or any other input device coupled to the non-invasive neuromodulation device. Automatic programs can be selected that vary in treatment time, such as 1-60 minutes. Longer programs ranging from 1-10 hours are also employed, such as on a sleeping patient.

The frequency of electrical signal outputs 18a and 18b is changed based on the series of frequencies in the treatment profile over the specified time periods. The series of frequencies alternates between high and low frequencies in a process referred to as "neural conditioning". In one or more embodiments, treatment comprises a plurality of intervals. In each interval, a starting frequency is first lowered, then elevated to an ending level lower than the starting frequency. Alternatively, a starting frequency is first raised, then lowered to an ending lever lower than the starting frequency.

In addition to the neural conditioning pattern described above, additional monotonically decreasing treatment cycles may be interspersed. In a monotonically decreasing treatment cycle, the frequency output commences at 150 KHz or greater, then is reduced over a time period T to a low of 50 Hz or lower without periodic increases in frequency output. These specific output patterns can be employed over various time periods from ten seconds to sixty minutes. The relationship between frequency and time may be a straight-line relationship or can be variable.

A treatment program comprises one or more treatment cycles. A treatment cycle follows a treatment profile which specifies a series of frequencies and time periods. The treatment profile can be used as a template with which to generate a treatment cycle, including treatment cycles of varying durations, frequencies, and intensities. Additional monotonically decreasing treatment cycles may be interspersed at any stage of a treatment profile.

A treatment program with a duration of T may include N treatment cycles. In one or more embodiments, each treatment cycle is generated from the same treatment profile. For each treatment cycle of a program, the treatment cycle can use the same frequencies specified in the treatment profile or modified frequencies based on the treatment profile. For example, a formula, such as a multiplier, can modify all frequencies, all high frequencies, or all low frequencies of a treatment profile. In one or more embodiments, a treatment program comprises 10 treatment cycles generated from the same treatment profile, wherein each treatment cycle is applied at a different intensity ranging from 10% to 100% of the total output energy specified in the treatment profile.

For a treatment program with a duration of T comprising N cycles, each treatment cycle may last for a duration of T/N. Alternatively, a treatment program may comprise treatment cycles of varying durations generated from different treatment profiles. The time durations specified in a treatment profile are scalable to a desired duration for a treatment cycle. In one or more embodiments, a treatment program spans both waking and sleeping hours, and different treatment profiles and/or durations are used while the patient is awake and when the patient is asleep.

In one or more embodiments, treatment programs are stored in memory, such as flash memory within the device. Stored treatment programs include preprogrammed treatment programs and user generated treatment programs. The device is configurable to generate a treatment program and treatment cycles based on user input, including treatment time, condition, desired intensity, or any other user selected factor.

**Example 1**

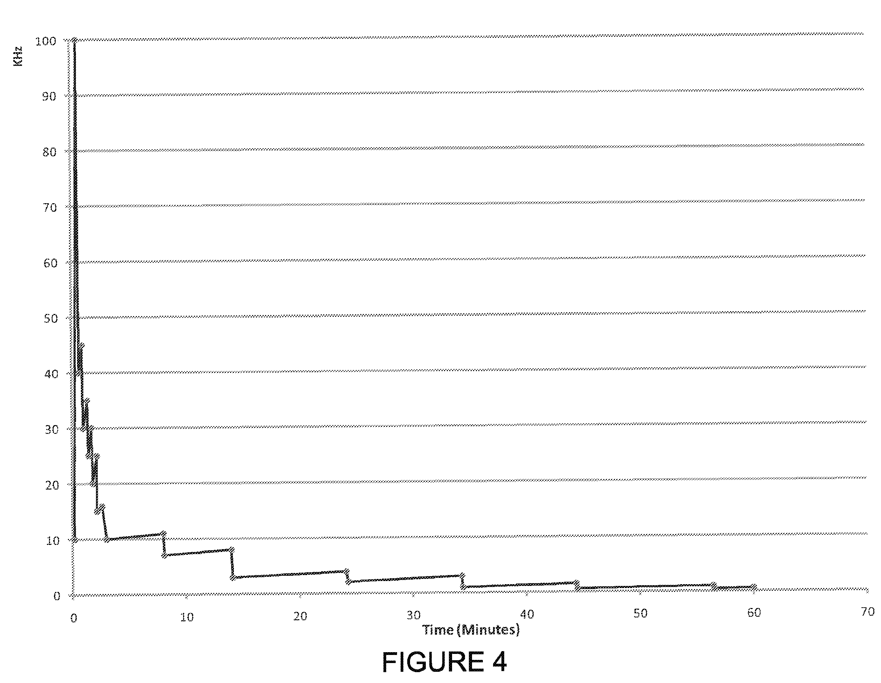

Table 1 comprises a series of frequencies and specified time periods for an exemplary treatment profile. A single frequency sweep changes from 10 KHz, up to 100 KHz, and after a series of changes, which ascend and descend in frequency output, finally cease at the lowest frequency, which can be 1Hz, or 0.1 Hz, or any lower number. In Example 1, the frequency is rapidly lowered at each decreasing step compared to the rate of increase at each increasing step. The entire frequency sweep occurs over a duration of 60 minutes. FIG. 4 presents a graph of the frequency sweep over a 60 minute treatment cycle generated from the treatment profile of Table 1, wherein the frequency is adjusted linearly over each time period. Alternatively, the frequency is adjusted non-linearly between frequencies.

**Table 1**

| **Start** **(Hz)** | **End (Hz)** | **Time** **(sec)** |
|---|---|---|
| 10,000 | 100,000 | 10 |
| 100,000 | 40,000 | 20 |
| 40,000 | 45,000 | 5 |
| 45,000 | 30,000 | 20 |
| 30,000 | 35,000 | 5 |
| 35,000 | 25,000 | 20 |
| 25,000 | 30,000 | 5 |
| 30,000 | 20,000 | 20 |
| 20,000 | 25,000 | 5 |
| 25,000 | 15,000 | 20 |
| 15,000 | 16,000 | 2 |
| 16,000 | 10,000 | 25 |
| 10,000 | 11,000 | 20 |
| 11,000 | 7,000 | 300 |
| 7,000 | 8,000 | 5 |
| 8,000 | 3,000 | 360 |
| 3,000 | 4,000 | 5 |
| 4,000 | 2,000 | 600 |
| 2,000 | 3,000 | 8 |
| 3,000 | 1,000 | 600 |
| 1,000 | 1,500 | 5 |
| 1,500 | 500 | 600 |
| 500 | 1,000 | 5 |
| 1,000 | 400 | 720 |
| 400 | 500 | 5 |
| 500 | 300 | 210 |

**Example 2:** Treatment of Clinical Depression

A treatment program comprising treatment cycles based on a treatment profile for clinical depression is described. Treatment of clinical depression includes treatment of symptoms, prevention, and controlling clinical depression.

Table 2 comprises a series of durations and frequencies in an exemplary treatment schedule to treat clinical depression. The treatment profile comprises 3 phases, corresponding to a high intensity phase, a moderate intensity phase, and a low intensity phase.

**Table 2: Exemplary treatment profile for clinical depression treatment profile**

| **Phase** | **duration** | **time** | **Frequency** **(KHz)** |
|---|---|---|---|
| 1 | | 0 | 10 |
| | 5 | 5 | 80 |
| | 5 | 10 | 30 |
| | 5 | 15 | 35 |
| | 5 | 20 | 20 |
| | 5 | 25 | 25 |
| | 5 | 30 | 12 |
| | 5 | 35 | 14 |
| | 5 | 40 | 8 |
| 2 | 5 | 45 | 9 |
| | 10 | 55 | 4 |
| | 5 | 60 | 4.5 |
| | 20 | 80 | 2.5 |
| | 5 | 85 | 2.8 |
| | 30 | 115 | 1 |
| 3 | 5 | 120 | 1.2 |
| | 70 | 190 | 0.8 |
| | 5 | 195 | 0.9 |
| | 80 | 275 | 0.6 |
| | 5 | 280 | 0.65 |
| | 80 | 360 | 0.35 |

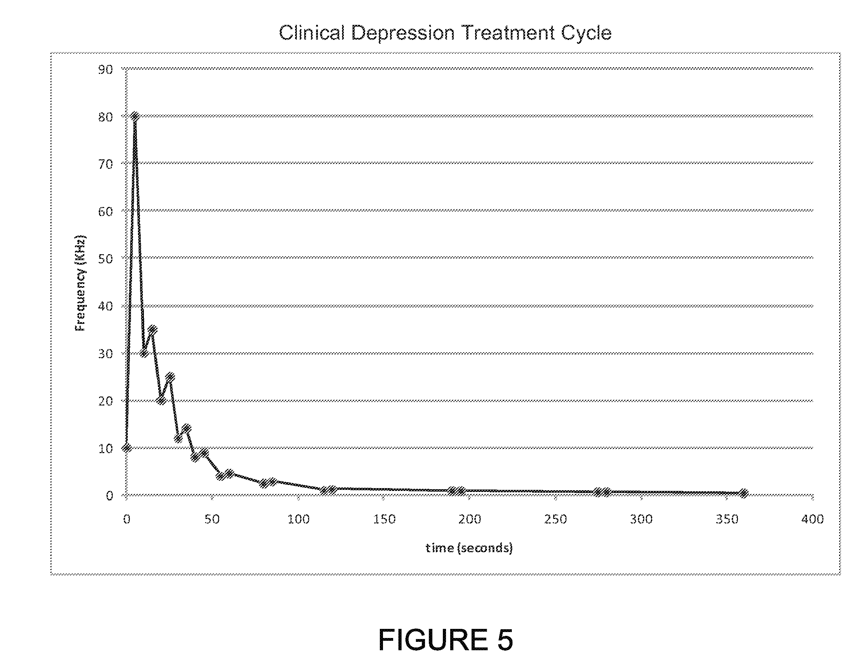

FIG. 5 presents a graph of the frequency sweep over a treatment cycle generated from the treatment profile of Table 2, wherein the frequency is adjusted linearly over each time period. Alternatively, the frequency is adjusted non-linearly between frequencies.

Embodiments of the device are portable devices preprogrammed with treatment programs comprising treatment cycles which are generated based on a treatment profile for treating clinical depression. For example, a device may provide treatment programs stored in memory varying in duration from 1 to 10 hours, each treatment program comprising 10 treatment cycles based on a treatment profile for treating clinical depression. In a treatment program, each treatment cycle is implemented at 10 different intensity levels. For example, a first treatment cycle is provided at an intensity of 1.5-2.5 volts. Treatment cycles 2-10 are provided at the following intensities: 3.5 - 5.5 V, 7.0 - 10.0 V, 10.0 - 13.5 V, 13.5 - 17.0 V, 15.8 - 19.5 V, 18.2 - 21.75 V, 20.25 - 23.0 V, 21.25 - 24.0 V, and 22.5 - 26.0 V. The duration of each treatment cycle and treatment program is scalable such that the length of the treatment program is longer or shorter than one hour.

In the previously described embodiments, phase 1, phase 2 and phase 3 of a treatment profile are repeated in order over 10 treatment cycles. Alternatively, a treatment program comprises a different combination of each phase in different orders. Table 3 provides ten exemplary preprogrammed treatment programs PROG 1-10 comprising different phases in different orders.

**Table 3: Phases comprising a treatment program**

| | Phase 1 | Phase 2 | Phase 3 |
|---|---|---|---|
| PROG 1 | 5 | 20 | 5 |
| PROG 2 | 5 | 17 | 7 |
| PROG 3 | 10 | 10 | 10 |
| PROG 4 | 6 | 12 | 10 |
| PROG 5 | 5 | 8 | 13 |
| PROG 6 | 4 | 7 | 14 |
| PROG 7 | 5 | 5 | 15 |
| PROG 8 | 4 | 4 | 16 |
| PROG 9 | 3 | 9 | 13 |
| PROG 10 | 1 | 10 | 13 |

**Example 3: Treatment of Cognitive Impairment**

A treatment program comprising treatment cycles based on a treatment profile for mild cognitive impairment and dementia is described. Treatment of mild cognitive impairment and dementia includes treatment of symptoms, prevention, and controlling clinical depression. Table 4 comprises a series of durations and frequencies in an exemplary treatment schedule to treat mild cognitive impairment and dementia. The term cognitive impairment is used broadly to cover both mild cognitive impairment and dementia.

**Table 4: Exemplary treatment profile for cognitive impairment**

| **Phase** | **duration** | **time** | **Frequency** **(KHz)** |
|---|---|---|---|
| 1 | | 0 | 10 |
| | 6 | 6 | 80 |
| | 6 | 12 | 60 |
| | 3 | 15 | 65 |
| | 6 | 21 | 45 |
| | 3 | 24 | 50 |
| | 10 | 34 | 30 |
| | 3 | 37 | 35 |
| | 23 | 60 | 15 |
| 2 | 5 | 65 | 17 |
| | 30 | 95 | 10 |
| | 5 | 100 | 12 |
| | 35 | 135 | 7 |
| | 10 | 145 | 8 |
| | 35 | 180 | 3 |
| 3 | 5 | 185 | 4 |
| | 55 | 240 | 2 |
| | 5 | 245 | 2.5 |
| | 55 | 300 | 1.5 |
| | 5 | 305 | 2 |
| | 55 | 360 | 0.4 |

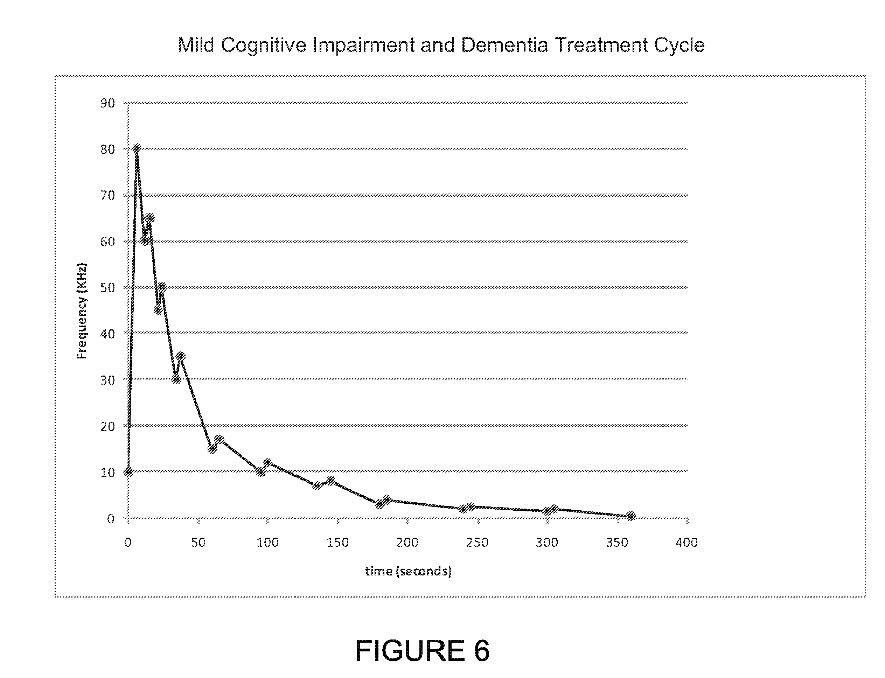

FIG. 6 presents a graph of the frequency sweep over a treatment cycle generated from the treatment profile of Table 3, wherein the frequency is adjusted linearly over each time period. Alternatively, the frequency is adjusted non-linearly with respect to time while changing frequencies over a time period.

Embodiments of the device are portable devices preprogrammed with treatment programs comprising treatment cycles which are generated based on a treatment profile for treating cognitive impairment. For example, a device may provide treatment programs stored in memory varying in duration from 1 to 10 hours, each treatment program comprising 10 treatment cycles based on a treatment profile for treating clinical depression. In a treatment program, each treatment cycle is implemented at 10 different intensity levels. For example, a first treatment cycle is provided at an intensity of 1.5-2.5 volts. Treatment cycles 2-10 are provided at the following intensities: 3.5 - 5.5 V, 7.0 - 10.0 V, 10.0 - 13.5 V, 13.5 - 17.0 V, 15.8 - 19.5 V, 18.2 - 21.75 V, 20.25 - 23.0 V, 21.25 - 24.0 V, and 22.5 - 26.0 V. The duration of each treatment cycle and treatment program is scalable such that the length of the treatment program is longer or shorter than one hour.

In the previously described embodiments, phase 1, phase 2 and phase 3 of a treatment profile are repeated in order over 10 treatment cycles. Alternatively, a treatment program comprises a different combination of each phase in different orders. Table 3 provides ten exemplary preprogrammed treatment programs.

**Example 4: Neurochemical Assays in Treated Humans**

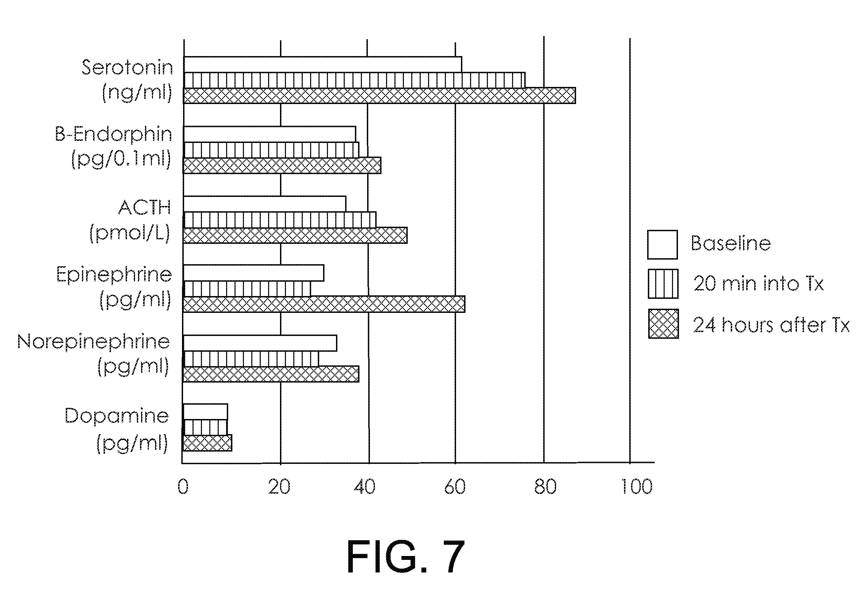

Patients were treated with a non-invasive neuromodulation device using electrode pads located at "Central Location" as described with reference to FIG. 3B. Blood samples were taken from subjects prior to treatment, after 20 minutes into treatment, and 24 hours after treatment had been completed. FIG. 7 presents a graph of neurochemical levels in patients before, during and 24 hours after treatment. As shown, serotonin levels (ng/ml), beta endorphin levels (pg/0.1ml), ACTH levels (pmol/1), epinephrine levels (pg/ml), norepinephrine levels (pg/ml), and dopamine levels (pg/ml) were elevated during and following treatment relative to baseline measurements prior to treatment. No other medical device has shown any evidence that it has the ability to increase a subject's neurochemical levels other than non-invasive neuromodulation devices developed from the same laboratory (Silverstone, Cella, Nusinow and Mossanen, 1998).

While the invention herein disclosed has been described by means of specific embodiments and applications thereof, numerous modifications and variations could be made thereto by those skilled in the art without departing from the scope of the invention set forth in the claims.

### REFERENCES

College's Committee on Obstetric Practice, December 2006: Opinion that pregnant women and those wishing to become pregnant refrain from taking the antidepressant Paxil because of the risk for birth defects. Obstetrics & Gynecology: December 2006.

Diem, S.J. et al, 2007. Use of antidepressants and rates of hip bone loss in older women. Arch. Intern. Medicine. 2007; 167: 1240-1245.

FDA Advisory Panel calls for suicide warnings over new antidepressants. Brit. Med. J. 2004. 328: 303.

Haney, E.M. et al, 2007. Association of low bone mineral density with selective serotonin reuptake inhibitor use by older men. Arch. Intern. Med. 2007; 167: 1246-1251.

Mann. T.I.J. and Silverstone, L.M. 1989. Clinical use of a new electronic dental anesthesia device. J. dent. Res., 69: 1027 (Special Issue).

O'Neill, M., Silverstone, L.M. and Halleck, M.E. 1990. Dental Anesthesia Apparatus. United States Patent and Trademark Office (USPTO), **Patent Number:** 4,924,880, May 15, 1990.

Panel calls for antidepressant warning. Reuters, December 13, 2006.

Saunders, A.M. and 13 other authors. 1993. Association of apolipoprotein E allele 4 with late-onset familial and sporadic Alzheimer's disease. Neurology, 43: 1467.

Silverstone, L.M. 1989. Electronic dental anaesthesia. Dental Practice, 27: 4-6

Silverstone, L.M. and Halleck, M.E. 1991. High frequency high intensity transcutaneous electrical nerve stimulator and method of treatment. United States Patent and Trademark Office (USPTO). ***Patent Number:*** 5,052,391, October 1, 1991.

Silverstone, L.M. 1992. Painless Electrology using Dial Away Pain 400. The Probe, 9. May 1-4

Silverstone, L.M. 1996. The use of a new non-invasive pain control system in the treatment of acute and chronic pain. Proceedings of the 3rd Congress, Int. Neuromodulation Soc., Orlando, Fl., March 6-19, 1996.

Silverstone, L. M., Cella, J. A., Nusinow, S. R. and Mossanen, A. 1998. Peripheral neurostimulation in the control of Essential Tremor. Proc. 4th Int. Cong., Int. Neuromodulation Society, Lucerne, Switzerland. Sept 1998.

Silverstone, L.M., Cella, J. A., Nusinow, S. R. and Mossanen, A. 1999 Non invasive neurostimulation in the control of familial essential tremor using the Synaptic neuromodulator. Conference Proceedings, International Functional Electrical Stimulation Society (IFES), Ed. Paul Meadows, May 1999.

Silverstone, L.M. 2000. Method and apparatus for treating chronic pain syndromes, tremor, dementia, and related disorders, and for inducing electroanesthesia using high frequency, high intensity, transcutaneous electrical nerve stimulation. United States Patent and Trademark Office (USPTO). ***Patent Number:*** 6,161,044, December 12, 2000.

Silverstone, L.M. 2003. Method and apparatus for treatment of viral diseases. United States Patent and Trademark Office. Patent Number: US 6.618,625, September 9, 2003.

Silverstone, L.M. 2006. Method and apparatus for treatment of viral diseases. Patent Number **1359971** was approved on July 2006 and has been issued in France, Germany, Italy, Spain and the United Kingdom.

Silverstone, L.M. 2007. Method and apparatus for treatment of viral diseases. Patent Number 2001298007 approved on May 25, 2007 and has been issued in Australia.

Silverstone, L.M. 2007. Method and apparatus for treatment of viral diseases. **Patent Number:** US 7,272,440 B2, Sept. 18, 2007

Silverstone, L.M. 2008. Biomedical Device Development: Keynote Address, 3rd Frontiers in Biomedical Devices Conference, American Society of Mechanical Engineers, June 16·20, 2008, Irvine, California.

Silverstone, L.M. 2008. The Development of Non·lnvasive Therapeutical Neuromodulation Medical Devices for the Treatment of Diseases of the Human Central and Peripheral Nervous Systems. Presentation at the Triennial Conference of the Chinese Society for Immunology and Virology, Hainan, China, September 19-21.2008.

Silverstone, L.M. 2009. New Advances in Non-Invasive Neuromodulation Treatment Devices. Invited Speaker, 4th Frontiers in Biomedical Devices Conference, American Society of Mechanical Engineers, June 8·, 2009, Irvine, California.

Weaver, F.M., Follett, K., Stern, M. and nineteen other authors. 2009. Bilateral deep brain stimulation vs best medical therapy for patients with advanced Parkinson disease. A randomized controlled trial. JAMA, Volume 301, 63-73, 2009.

Wozniak, M.A., Mee, A.P., and Itzhaki, R.F. 2009. Herpes simplex virus type I DNA is located within Alzheimer's disease amyloid plaques. The Journal of Pathology, Volume 217, 131 - 138, 2009.

## Claims

1. An apparatus for providing non-invasive neuromodulation for use in treating a neurological disorder in a patient, the apparatus comprising:
means for generating an electrical signal comprising a stimulation frequency; and
means for applying said electrical signal transcutaneously to a patient over at least one treatment cycle comprising a plurality of intervals, wherein each interval comprises:
increasing said stimulation frequency to a high frequency; and
decreasing said stimulation frequency to a low frequency,
wherein said electrical signal is transmitted by Aδ and C nerve fibers.

2. The use of claim 1, further comprising means for applying at least one additional monotonically decreasing treatment cycle, wherein said at least one additional monotonically decreasing treatment cycle comprises a monotonically decreasing electrical signal beginning at about 150 KHz or greater, wherein said monotonically decreasing electrical signal is reduced over a time period to about 50 Hz or lower without periodic increases in frequency, wherein said time period is from about ten seconds to about sixty minutes.

3. The use of claim 1 or 2, wherein said neurological disorder is selected from clinical depression, and cognitive impairment.

4. The use of any preceding claim, wherein said stimulation frequency is variable between .01Hz and 1,000,000 Hz.

5. An electrical stimulation device comprising a first electrode and a second electrode, wherein said first electrode and said second electrode are configured to apply an electrical stimulation transcutaneously to a patient at a variable stimulation frequency, for use in treating a neurological disorder in a patient by non-invasive neuromodulation, said use comprising the steps of obtaining a treatment profile comprising a series of first frequency settings A₀, A₁, A₂, ..., A_{N}, a series of second frequency settings B₁, B₂, ...,B_{N} and a series of time durations t₁, t₂, ..., t_{2N,} wherein Aᵢ ≤ Aᵢ₋₁ and Bᵢ ≤ Bᵢ₋₁;
applying at least one treatment cycle based on said treatment profile, wherein applying a treatment cycle comprises:
applying said electrical stimulation transcutaneously to a patient, wherein said variable stimulation frequency is set to a frequency A₀;
increasing and decreasing the variable stimulation frequency sequentially over N intervals, wherein increasing and decreasing comprises:
(a) steadily changing the variable stimulation frequency to said second frequency setting B₁ over time duration t₁,
(b) steadily changing the variable stimulation frequency to A₁ over time duration t₂,
(c) repeating steps (a) and (b) sequentially for all first frequency settings and all second frequency settings.

6. The use of claim 5, wherein Aᵢ₋₁ ≤ Bᵢ or Aᵢ₋₁ > Bᵢ

7. The use of claim 5 or 6, wherein said variable stimulation frequency is between .01Hz and 1,000,000 Hz.

8. The use of claim 5, 6 or 7, further comprising applying at least one additional monotonically decreasing treatment cycle, wherein said at least one additional monotonically decreasing treatment cycle comprises a monotonically decreasing electrical signal beginning at about 150 KHz or greater, wherein said monotonically decreasing electrical signal is reduced over a time period to about 50 Hz or lower without periodic increases in frequency, wherein said time period is from about ten seconds to about sixty minutes.

9. The use of any of claims 5 to 8, wherein said treatment profile is designed for the treatment of clinical depression and/or cognitive impairment.

10. The use of any of claim 5 to 9, further comprising:
obtaining a second treatment profile, and
applying at least one treatment cycle based on said second treatment profile.

11. The use of any of claim 5 to 10, further comprising applying a modified treatment cycle based on said treatment profile, wherein modified durations t'₁, t'₂, ..., t'_{2N} are scaled based on said durations t₁, t₂, ..., t_{2N} .

12. The use of any of claim 5 to 11, further comprising applying a modified treatment cycle based on said treatment profile, wherein a modified series of first frequency settings and a modified series of second frequency settings are scaled based on said series of first frequency settings and said series of second frequency settings and/or said at least one treatment cycle comprises at least two treatment cycles and said at least two treatment cycles are applied at different voltage intensities.

13. A device for providing non-invasive neuromodulation to treat a neurological disorder, comprising:
a housing;
at least two electrodes configured to apply an electrical stimulation at a variable stimulation frequency;
a power source;
a treatment program stored in a memory comprising a treatment profile, said treatment profile comprising a series of first frequency settings A₀, A₁, A₂, ..., A_{N}, a series of second frequency settings B₁, B₂, ...,B_{N} and a series of time durations t₁, t₂, ..., t_{2N}, wherein Aᵢ ≤ Aᵢ₋₁ and Bᵢ₋₁ ≤ Bᵢ₋₁;
an electronic circuit positioned inside said housing and coupled to said power source,
said memory and said at least two electrodes, wherein said electronic circuit is configured to execute said treatment program, wherein executing said treatment program comprises:
generating said electrical stimulation;
setting said variable stimulation frequency to A₀;
increasing and decreasing the variable stimulation frequency sequentially over N intervals, wherein increasing and decreasing comprises
(a) steadily changing the variable stimulation frequency to said second frequency setting B₁ over time duration t₁,
(b) steadily changing the variable stimulation frequency to A₁ over time duration t₂, and
(c) repeating steps (a) and (b) sequentially for all first frequency settings and all second frequency settings.

14. The device of claim 13, wherein said treatment profile is designed for the treatment of clinical depression and/or cognitive impairment.

15. The device of claim 13 or 14, wherein said device is a portable device, said power source is a battery, and said at least two electrodes are located on a contact surface coupled with said housing, wherein said contact surface is configured to be applied transcutaneously to a patient.
